# FASCICULE DE BREVET EUROPEEN

(11) **EP 2 157 931 B1**
(45) Date de publication et mention de la délivrance du brevet: **23.02.2011**
(21) Numéro de dépôt: 08826611.9
(22) Date de dépôt: 19.06.2008
(51) Int. Cl.: A61B 19/00

(54) **PLATEFORME ROBOTISEE MULTI-APPLICATIVE POUR LA NEUROCHIRURGIE ET PROCEDE DE RECALAGE**
ROBOTER-PLATTFORM MIT MEHREREN ANWENDUNGEN FÜR DIE NEUROCHIRURGIE UND RÜCKSETZUNGSVERFAHREN DAFÜR
MULTI-APPLICATION ROBOTISED PLATFORM FOR NEUROSURGERY AND RESETTING METHOD

(30) Priorité: 19.06.2007 FR 0704350
(43) Date de publication de la demande: 03.03.2010
(73) Titulaire: Medtech S.A., 34090 Montpellier (FR)
(72) Inventeur: NAHUM, Bertin, F-34670 Baillargues (FR); BLONDEL, Lucien, F-34000 Montpellier (FR)
(74) Mandataire: Rhein, Alain
(86) Numéro de dépôt international: PCT/FR2008/000860
(87) Numéro de publication internationale: WO 2009/013406

(56) Documents cités:
- WO-A-98/38908
- WO-A-03/043515
- WO-A-2007/113713
- FR-A- 2 871 363
- US-A- 6 033 415
- US-A1- 2004 111 183
- US-A1- 2004 243 109
- US-A1- 2006 100 642
- US-A1- 2007 106 306

## Description

### Domaine technique.

La présente invention est du domaine des matériels utilisés en médecine et plus spécifiquement en neurochirurgie. Elle concerne plus particulièrement une plateforme robotisée multi-applicative pour la neurochirurgie et le procédé de mise en oeuvre associé.

### État de la technique antérieure.

On sait que la pratique de la neurochirurgie nécessite l'utilisation d'un nombre croissant d'équipements et de matériels chirurgicaux dédiés.

Ainsi sont utilisés des cadres stéréotaxiques notamment pour les biopsies tumorales ou le positionnement précis d'électrodes de stimulation. Un inconvénient de ces cadres est qu'ils ne se prêtent pas ou peu à la chirurgie ouverte. Outre leur encombrement, un autre inconvénient réside essentiellement dans le fait qu'ils nécessitent un ancrage ferme dans les os de la boîte crânienne.

On connaît aussi des robots qui peuvent être utilisés en remplacement des cadres stéréotaxiques.

On connaît également de l'état de la technique des systèmes de neuro-navigation offrant une solution pertinente pour la chirurgie ouverte. Ces systèmes permettent le repérage de structures anatomiques à partir d'une imagerie pré-opératoire donnée par un tomodensitomètre, par un appareil d'imagerie par résonance magnétique (IRM) ou autre et d'un système de localisation en trois dimensions, comprenant par exemple plusieurs sources lumineuses attachées à l'instrument, émettant dans le domaine de l'infra rouge.

Ces systèmes comprennent de plus une ou plusieurs cameras aptes à percevoir l'infrarouge et à émettre un signal lequel sera traité par un calculateur approprié apte à calculer les données de positions et orientations spatiales de l'instrument chirurgical telles que la position de la pointe de ce dernier.

Typiquement en préalable à la réalisation de l'imagerie, le crâne du patient est équipé de marqueurs radio-opaques, sous forme de pastilles, prévus pour être apposés à même la peau. Les images numériques obtenues sont transférées dans un bloc mémoire du calculateur. À l'aide de l'instrument chirurgical ou d'un instrument spécifique le chirurgien amène la pointe de cet instrument au contact de chacun des marqueurs radio-opaques. Ainsi, la position de l'instrument peut être repérée par rapport aux images médicales numérisées précédemment obtenues. De cette manière, au cours de l'acte chirurgical notamment, l'image de l'instrument et sa position peuvent être superposées aux images numériques en vue d'un affichage conjoint sur un écran de visualisation.

Les systèmes de neuro-navigation sont notamment utilisés pour le repérage et la résection de tumeurs cérébrales.

On connaît aussi des microscopes chirurgicaux utilisés comme outils de visualisation lors d'actes neurochirurgicaux à ciel ouvert (ex. : cortectomie).

Les applications chirurgicales décrites ci-dessus et les équipements associés représentent une partie importante de la pratique routinière en neurochirurgie.

Cependant, la pluralité des équipements et leur spécificité à un type d'application neurochirurgicale sont pénalisantes dans la gestion logistique hospitalière, et contradictoires avec les objectifs de flexibilité et de polyvalence du bloc opératoire.

Un autre inconvénient spécifique à la neuro-navigation réside dans les possibilités d'erreurs de recalage entre le modèle numérique établi, le patient, et l'outil. Ceci tient essentiellement au fait que les pastilles radio-opaques sont apposées sur la peau qui demeure un organe mouvant, et non implantées de manière fixe dans les os crâniens.

Pour la mise en place de ces marqueurs radio-opaques, le praticien cherche cependant à éviter toute procédure invasive en dépit du risque de perte de précision du recalage dû au déplacement involontaire d'un des marqueurs.

On connaît aussi de l'état de la technique un dispositif robotisé de guidage pour outil chirurgical. Un tel outil est notamment décrit dans la demande de brevet FR 2 871 363. Ce dispositif robotisé comprend un bras robot, des moyens de collecte de repères anatomiques à l'aide du bras robot, des moyens de traitement de ces repères anatomiques et des moyens de positionnement automatique d'un instrument de guidage d'un outil chirurgical, cet instrument de guidage étant porté par le bras robot.

Ce dispositif de guidage n'est pas doté de moyens de capture d'images du champ opératoire ni de moyens spécifiques de visualisation du champ opératoire.

Ce dispositif ne répond pas au but poursuivi par la présente invention.

### Exposé de l'invention.

La présente invention vise à résoudre les problèmes sus évoqués en proposant d'une part, une solution multi-applicative se substituant à l'ensemble des dispositifs listés ci-dessus et d'autre part un procédé visant à un meilleur recalage du modèle numérique de la zone anatomique à traiter avec le patient et l'outil chirurgical.

À cet effet l'invention telle que définie dans la revendication 1 propose une plateforme robotisée multi-applicative pour la neurochirurgie caractérisée essentiellement en ce qu'elle comporte :
- une console de planification intégrant des moyens de traitement capables notamment de recevoir et traiter des images numériques,
- un bras robot positionneur comportant plusieurs segments de bras dont un est terminal et proximal et l'autre terminal et distal, les dits segments étant liés les uns autres par des articulations et le segment distal terminal de bras comportant un organe récepteur agencé pour recevoir des outils instruments et autres, ledit bras robot étant piloté par la console de planification,
- au moins un moyen de capture d'images vidéo apte à capturer des images de la zone anatomique à traiter, ce dit moyen de capture pouvant être connecté électriquement aux moyens de traitement que comporte la console de planification, et ce dit moyen de capture étant apte à être positionné et fixé de manière amovible à l'organe récepteur que comporte le segment distal terminal de bras,
- des outils, instruments et autres adaptés pour être positionnés et fixés de manière amovible à l'organe récepteur du segment distal terminal de bras,
- des moyens de visualisation des images pré-opératoires et per-opératoires, les dits moyens étant électriquement connectés à la console de planification pour recevoir de cette dernière des signaux vidéo propres aux images à visualiser, et/ou au moyen de capture d'image.

Selon une autre caractéristique de l'invention, le bras robot positionneur présente au moins six degrés de liberté à savoir trois translations et trois rotations grâce à quoi l'outil ou l'instrument et autre qu'il porte peut être positionné et orienté dans l'espace de toutes les façons possibles.

Selon une autre caractéristique de l'invention, le bras robot comporte un capteur d'effort et est adapté à fonctionner selon un mode dans lequel un utilisateur a la capacité de déplacer le bras robot manuellement en le saisissant par sa partie terminale. Le bras robot fonctionne alors en mode coopératif.

Selon une autre caractéristique de l'invention, la console de planification est équipée d'un écran de contrôle et d'une interface de communication adaptée à recevoir d'un utilisateur des paramètres de planification opératoire.

Grâce à ces dispositions, les moyens de traitement peuvent tenir compte des paramètres de planification opératoire pour commander la trajectoire du robot positionneur et plus particulièrement la trajectoire de l'outil ou de l'instrument, ou du moyen de capture d'image qu'il porte.

Ainsi, le point d'entrée crânien et le point cible dans la masse cervicale, par exemple, peuvent être fournis à la plateforme grâce à une interface graphique simple et conviviale.

L'interface de communication peut, par exemple, prendre la forme d'un clavier, d'une interface tactile et/ou d'un dispositif de pointage du genre souris par exemple.

Selon des caractéristiques particulières de l'invention, les moyens de traitement sont adaptés à défmir chaque trajectoire grâce à des calculs tridimensionnels réalisés à partir des paramètres de planification opératoire et des coordonnées spatiales des éléments de recalage.

Selon une autre caractéristique de l'invention, les outils comprennent au moins un palpeur avec ou sans contact et/ou au moins une sonde ultrasons, et/ou au moins un télémètre.

Selon une autre caractéristique de l'invention, le palpeur est un instrument de pointage mécanique adapté à être fixé de manière amovible sur le bras robot.

En mode coopératif, l'utilisateur peut pointer un élément sur la tête du patient en déplaçant manuellement l'instrument de pointage et en venant au contact de la cible. Un tel palpeur permet à l'utilisateur d'acquérir par exemple les positions de points anatomiques remarquables ou les positions de marqueurs radio-opaques ou les positions d'une multitude de points au contact de la peau du patient afin d'en obtenir une surface par reconstruction.

Selon l'invention, le palpeur est un télémètre laser.

À partir des informations des codeurs du bras robot, de la géométrie du palpeur et de la mesure de distance fournie par le module optique, le système pourra calculer la position tridimensionnelle du point de l'objet intersecté par le faisceau laser directement dans le système de coordonnées du bras robot.

Dans ce cas, le palpeur fournit une solution de pointage virtuel sans contact. De la même manière qu'avec l'instrument de pointage mécanique, un tel palpeur permet à l'utilisateur d'acquérir les positions de points anatomiques remarquables, de marqueurs radio-opaques ou d'une multitude de points au contact de la peau du patient afin d'en obtenir une surface par reconstruction.

Selon une autre caractéristique de l'invention, l'un au moins des outils est constitué par un guide tubulaire ou canon de guidage.

Grâce à ces caractéristiques, la plateforme robotisée équipée d'un guide tubulaire ou d'un canon monté en fixation sur l'organe récepteur du bras positionneur, peut être utilisée en tant que cadre stéréotaxique, le canon maintenu en position spatiale fixe par le bras robot positionneur offrant un guidage axial pour un foret de perçage, pour une électrode, pour une aiguille et autre instruments et moyens utilisables dans le cadre de la neurochirurgie stéréotaxique.

Lorsque la mise en correspondance entre les images pré-opératoires (scanner, IRM ou autre modalité) et la position du patient est effectuée, le système connait la position du ou des instruments portés par le bras robot.

Cet instrument positionné par le bras robot en accord avec la planification peut être un pointeur laser ou autre type de pointeur. Le pointeur laser permet alors de cibler sur le patient une structure anatomique identifiée sur l'imagerie pré-opératoire. En mode coopératif (tel que défini ' ci-dessus), l'utilisateur a la capacité de pointer une cible sur la tête du patient en saisissant, par sa partie terminale, le bras robotisé positionneur équipé de pointeur laser, et en le déplaçant manuellement. La direction pointée est représentée sur les images pré-opératoires de la console de planification.
La plateforme, objet de la présente invention, se substitue alors avantageusement à un système de neuro-navigation.

L'avantage d'un tel bras robot est qu'il peut maintenir en position le pointeur, ce qui n'est pas le cas des systèmes de neuro-navigation actuels lorsque le pointeur est tenu manuellement.

Selon une autre caractéristique de l'invention, l'un au moins des outils est constitué par un instrument chirurgical. De cette façon le geste chirurgical sera effectué non plus par le chirurgien mais par le bras robot en accord avec la planification.

Le moyen de capture d'images est prévu pour être fixé de manière amovible en extrémité du bras robot. Ce moyen de capture d'images comporte au moins une caméra vidéo du type numérique par exemple.

Grâce à ce moyen de capture d'images, l'utilisateur peut visualiser sur le patient une zone d'intérêt par exemple une zone identifiée sur l'imagerie pré-opératoire. En mode coopératif, tel que défini ci-dessus, l'utilisateur a la capacité de visualiser une zone de son choix sur la tête du patient en saisissant le bras positionneur par sa partie terminale et en le déplaçant manuellement. La zone visualisée est représentée sur les images pré-opératoires de la console de planification.

Les moyens de visualisation des images issues de la caméra peuvent par exemple, non limitativement, être un écran du type 2D et/ou un casque du type 2D ou bien encore préférentiellement du type 3D si les techniques de stéréovision sont mises en oeuvre.

La plateforme, objet de la présente invention, se substitue alors avantageusement à un microscope chirurgical navigué. Le flux vidéo délivré par le moyen de capture d'images peut être transmis simultanément à l'écran de la console de planification. Ainsi, le chirurgien et le reste de son équipe visualisent la même image vidéo de la zone anatomique pendant l'opération. Ils peuvent être transmis à un autre moyen de visualisation mais aussi, de manière simultanée, à cet autre moyen de visualisation et à l'écran de la console de planification.

Au lieu de déplacer manuellement les moyens de capture d'images en saisissant le bras robot en mode coopératif, l'utilisateur peut également piloter les déplacements du bras robot par le biais d'une boîte de commande. Cette même boîte de commande permet le réglage de la ou des caméra(s) du moyen de capture d'image, notamment le niveau de zoom et la distance de mise au point. Cette boîte de commande pourra comporter des boutons de commande et/ou au moins une manette de commande.

Alternativement, le positionnement du moyen de capture d'images en mode coopératif, c'est-à-dire en saisissant par sa partie terminale le bras positionneur équipé de ce moyen de capture et en le déplaçant manuellement, peut se faire sans correspondance spatiale au niveau de la console de planification. La plateforme objet de la présente invention, est alors l'équivalent à un simple microscope chirurgical.

Selon une autre caractéristique de l'invention, le moyen de capture d'images du champ opératoire et autre zones anatomiques pertinentes, comporte une paire de caméras stéréoscopiques par exemple du type numérique afin d'acquérir deux images vidéos stéréoscopiques de la zone anatomique à traiter et pouvoir restituer une vue 3D de la zone grâce à un système de visualisation d'images stéréoscopiques faisant partie de l'invention.

L'avantage de cette méthode est de restituer la perception du relief au chirurgien, améliorant ainsi la qualité de son geste chirurgical.

Selon une autre caractéristique de l'invention, le moyen de capture d'images intègre un module optique adapté à être interfacé à un câble optique lui-même connecté à une source de lumière froide. Ainsi, le dispositif éclaire la zone anatomique à traiter en même temps qu'il en acquiert un flux vidéo.
Un inconvénient connu de certains microscopes opératoires est qu'ils nécessitent un éclairage puissant de la zone anatomique afin que l'image transmise dans les binoculaires ait une luminosité suffisante. L'utilisation de caméras vidéo du type numériques est un avantage certain car elles ne nécessitent pas un éclairage puissant. Un éclairage ambiant peut alors suffire pour une vision correcte de la zone anatomique.

Selon une autre caractéristique de l'invention, le moyen de capture d'images intègre deux modules lasers qui projettent des faisceaux lasers visibles. Ces faisceaux lasers convergent en un point qui peut être réglé pour être le point d'intersection des deux axes optiques des caméras stéréoscopiques.
Ces modules lasers convergents apportent un avantage en cours d'utilisation car ils indiquent la zone de travail optimale pour la perception du relief. Ils ont également un avantage en production pour faciliter le centrage des deux caméras stéréoscopiques sur le même point et déterminer la géométrie de l'outil moyen de capture d'images.

Selon une autre caractéristique de l'invention, le moyen de capture d'images intègre un module laser central aligné sur l'axe optique central de la paire de caméras stéréoscopiques. Ce faisceau laser visible, matérialise l'axe dans lequel l'image vidéo de la zone anatomique est acquise.

Selon une autre caractéristique de l'invention, le module laser central est un télémètre laser apte à mesurer la distance entre sa face externe et l'objet le plus proche pointé par le faisceau laser.

Selon une autre caractéristique de l'invention, le moyen de capture d'images intègre un système mécanique de rotation autour de son axe optique. L'utilisateur peut ainsi faire tourner le moyen de capture d'images à l'aide d'une poignée afin d'orienter l'image vidéo de la zone anatomique selon les besoins de son acte chirurgical.

Selon une autre caractéristique, l'invention intègre un pointeur mécanique équipé d'au moins un marqueur visible, connu en soi, adapté à être repéré sur les images vidéo acquises par le moyen de capture d'images. La position de la pointe du pointeur peut alors être calculée par triangulation en identifiant le(s) marqueur(s) sur les deux images stéréoscopiques.
Ce calcul nécessite la calibration préalable de chacune des deux caméras (paramètres intrinsèques) ainsi que la calibration du système stéréoscopique (position et orientation d'une caméra par rapport à l'autre). L'avantage de cette solution est que la paire de caméras stéréoscopiques est située dans une région proche de la zone anatomique à traiter et qu'on s'affranchit ainsi du problème de « ligne de vue » commun aux systèmes actuels de neuro-navigation par localisation optique.

Lorsque la mise en correspondance entre les images pré-opératoires (scanner, IRM ou autre modalité) et la position du patient est effectuée, le système connait la position du moyen de capture d'images porté par le bras robot. Le système peut se substituer alors avantageusement à un microscope combiné à un système de neuro-navigation. Le point d'intérêt affiché sur la zone anatomique et sur les images pré-opératoires de la console de planification peut être par exemple le point d'intersection des faisceaux lasers convergents et/ou le point d'impact du faisceau laser central et/ou le point au contact du pointeur mécanique équipé de marqueurs visibles.

Selon une autre caractéristique de l'invention, est prévu un système de visualisation d'images stéréoscopique du type trois dimensions.

Selon une caractéristique de l'invention, le système de visualisation d'images stéréoscopiques est composé de deux écrans adaptés à afficher deux images vidéo provenant de sources différentes. Ces écrans peuvent être avantageusement montés sur un casque ou sur des lunettes afin que le chirurgien conserve les mains libres pour son geste chirurgical.

Ce système de visualisation peut être utilisé durant l'étape de planification du geste sur la console pour afficher une vue réaliste des objets virtuels 3D ; par exemple, le modèle numérique du patient établi à partir des images pré-opératoires, ou des objets virtuels de planification tel qu'un point cible, un point d'entrée, une trajectoire rectiligne, une surface d'intérêt, un volume d'intérêt, etc.

Le chirurgien manipule ainsi directement des informations sous forme tridimensionnelles contrairement aux systèmes de neuro-navigation existants qui ne disposent que d'un écran affichant des informations sous forme bidimensionnelle.

Le dispositif peut également se substituer avantageusement à un microscope opératoire en affichant par le système de visualisation les images vidéo provenant du moyen de capture d'images. Le chirurgien peut alors opérer dans la position qu'il considère optimale pour son geste chirurgical.

Une fois que la mise en correspondance entre les images pré-opératoires (scanner, IRM ou autre modalité) et la position du patient au bloc opératoire est effectuée, la console de planification connait la position du moyen de capture d'images porté par le bras robot. Avec la connaissance à priori du modèle projectif des caméras, il est possible de superposer des images virtuelles sur les images réelles de façon à visualiser un élément défini de la zone anatomique ciblée. Cet élément peut être par exemple une tumeur dans le cas d'une résection tumorale, ou un point cible, ou un point d'entrée, ou une trajectoire, ou un instrument, ou encore une zone anatomique d'intérêt. Le système de visualisation stéréoscopique affiche alors des images vidéo de la zone anatomique, augmentées d'éléments virtuels de la planification. Est assurée alors la fonction de réalité augmentée.

La réalité augmentée permet de fournir des informations précieuses au chirurgien pendant qu'il opère. C'est une fonction très avantageuse puisqu'elle évite au chirurgien de regarder alternativement la zone qu'il opère et les images pré-opératoires affichées sur un écran et de faire mentalement la correspondance entre ces informations. Toutes les informations sont projetées de manière superposées sur le même écran. Le système de visualisation stéréoscopique peut afficher notamment des informations sous forme textuelle, par exemple des distances ou des volumes.

Ces caractéristiques confèrent à la plateforme un caractère multi-applicatif qui répond aux besoins des centres hospitaliers en termes de gestions logistiques et de maintenance et répond aux objectifs de flexibilité et de polyvalence du bloc opératoire.

La présente invention à également pour objet un procédé visant à augmenter la précision du recalage entre la zone anatomique à traiter, son modèle numérique et un bras robotisé.

À cet effet, le procédé selon l'invention tel que définie dans la revendication 17 consiste:
- en l'acquisition, antérieurement à l'intervention neurochirurgicale, de premières images numériques de la zone à traiter et au transfert de ces images numériques vers la console de planification par le biais d'un réseau ou d'un support physique en vue d'y être enregistrées et traitées,
- en l'acquisition en per-opératoire, à l'aide d'un instrument de balayage porté par l'organe récepteur du segment distal terminal de bras robotisé, de secondes images numériques d'une partie pertinente d'une région corporelle du patient apparaissant déjà sur les premières images numériques, et au transfert de ces secondes images numériques vers la console de planification en vue d'y être enregistrées et traitées,
- en la construction d'un premier modèle numérique en trois dimensions à partir des premières images numériques, ledit modèle faisant apparaître la région corporelle pertinente du patient,
- en la construction d'un second modèle numérique en trois dimensions à partir des secondes images numériques, faisant toujours apparaître la région corporelle pertinente du patient,
- et en la mise en correspondance des premier et second modèles par superposition des représentations de la région corporelle pertinente apparaissant sur l'un et l'autre modèle.

Un tel procédé s'affranchit ainsi de la présence de marqueurs radio-opaques et augmente le degré de précision du recalage entre le modèle pré-opératoire, le patient et le bras robotisé.

### Description sommaire des figures et des dessins.

D'autres avantages et caractéristiques de l'invention apparaîtront à la lecture de la description d'une forme préférée de réalisation donnée à titre d'exemple non limitatif en se référant aux dessins annexés en lesquels :

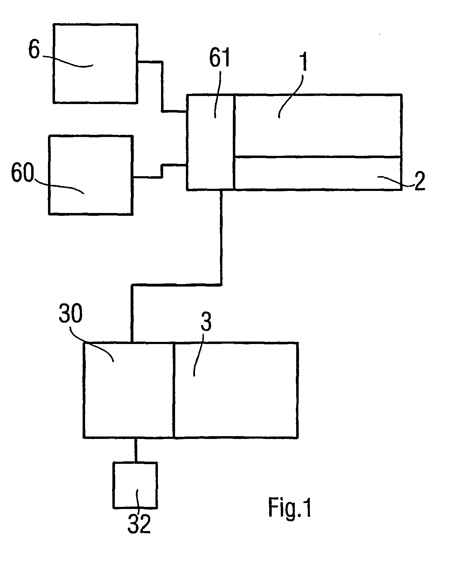

- la figure 1 est une vue synoptique de la plateforme selon l'invention,

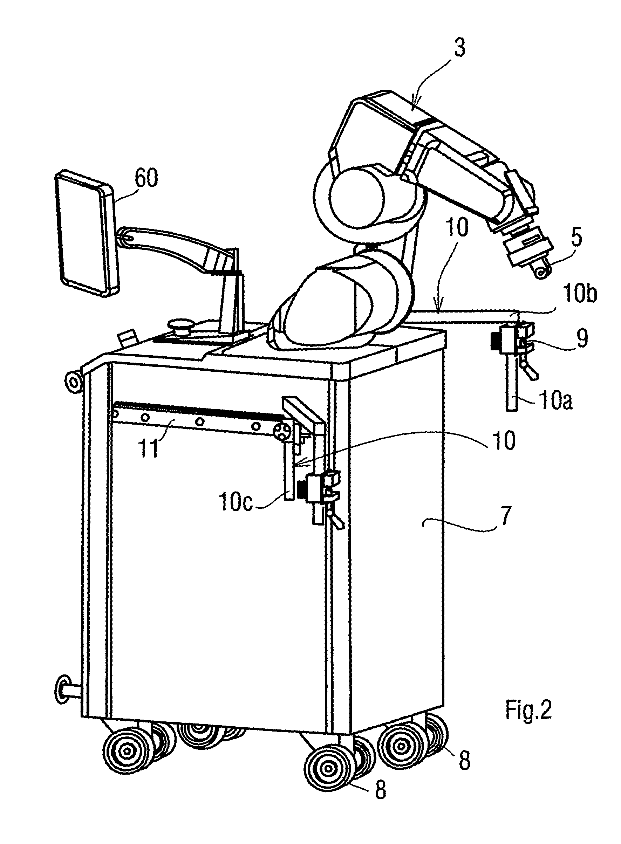

- la figure 2 est une vue en perspective d'une plateforme selon l'invention,

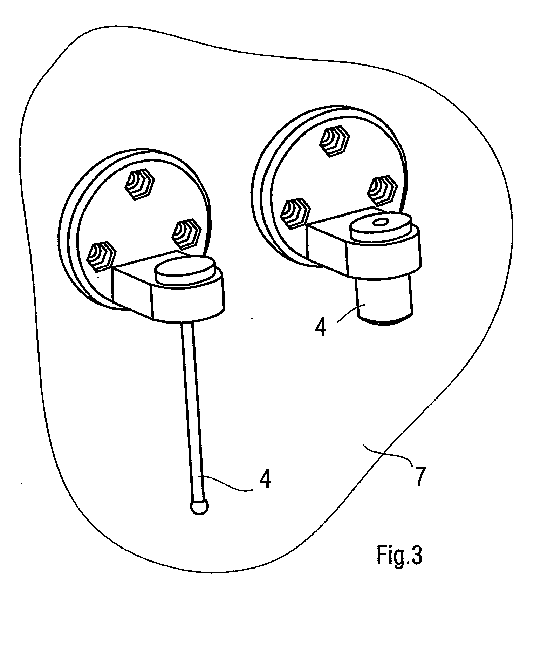

- la figure 3 est une vue de détail de la plateforme selon l'invention,
- la figure 4 est une vue schématique montrant d'un exemple de réalisation d'un moyen d'immobilisation du caisson par rapport au sol, ce moyen étant en position déployée,
- la figure 5 est une vue schématique d'un exemple de réalisation d'un moyen de fixation du caisson à une têtière prévue pour recevoir la tête du patient à opérer,
- la figure 6 est une vue schématique d'un exemple de réalisation d'un palpeur sans contact porté par le bras robot,
- la figure 7 est une vue schématique d'un exemple de réalisation d'un moyen de capture d'images,
- la figure 8 est une vue schématique d'un exemple de réalisation d'un moyen de visualisation du type trois dimensions,
- la figure 9 est une vue schématique d'un exemple de réalisation d'un pointeur mécanique équipé d'un marqueur visible.

### Meilleure manière de réaliser l'invention

Telle que représentée, la plateforme robotisée multi-applicative pour la neurochirurgie, selon l'invention, comprend une console 1 de planification, qui peut être embarquée, intégrant des moyens de traitement 2 capables notamment de recevoir et traiter des images numériques, un bras robotisé positionneur 3 comportant plusieurs segments de bras dont un est terminal et proximal et l'autre terminal et distal, les dits segments étant liés les uns autres par des articulations et le segment distal terminal de bras comportant un organe de récepteur 5 agencé pour recevoir en fixation de manière amovibles des outils 4, instruments et autres, ledit bras robot étant piloté par la console de planification 1.

La plateforme intègre également une série d'outils et éventuellement d'instruments chirurgicaux adaptés pour être positionnés et fixés de manière amovible à l'organe récepteur 5 du segment distal terminal de bras comme exposé précédemment, ainsi qu'un moyen 14 de capture d'images vidéo du champ opératoire et des moyens de visualisation 6 des images pré-opératoire et per-opératoire. Ces moyens visualisation sont électriquement connectés à la console de planification pour recevoir de cette dernière des signaux vidéos relatifs aux images à visualiser, et/ou à un moyen 14 de capture d'images vidéo telle qu'une caméra.

Les moyens de visualisation pourront comprendre un moyen de visualisation du type trois dimensions.

La plateforme sera également équipée d'un écran de contrôle 60 et d'une interface de communication 61 adaptée à recevoir d'un utilisateur des paramètres de planification opératoire.

Au bras robot positionneur 3 sont associés une unité centrale et une interface d'entrée informatique de données qui peuvent faire partie de l'interface de communication précitée.

Le bras robotisé positionneur 3, par son segment proximal terminal, est fixé à une tourelle d'orientation installée fixement sur la partie supérieure d'un caisson parallélépipédique 7. Ce caisson abrite une électronique, propre notamment à la commande du bras 3.

Entre les segments de bras, du bras robot positionneur 3 sont prévus des articulations par exemple aux nombre de six comportant des moteurs et des codeurs incrémentaux associés, au niveau de chaque articulation, à l'axe ou à chacun des axes de pivotement que définit cette dernière. Chaque moteur est apte à entraîner en pivotement deux segments contigus l'un par rapport à l'autre et chaque codeur incrémental associé est apte à donner une information relative à la position angulaire de l'un de ces segments par rapport à l'autre. Les articulations permettent de positionner l'outil terminal, l'instrument et autre, tant en position (trois degrés de liberté) qu'en orientation (trois degrés de liberté). Les valeurs angulaires mesurées par les codeurs incrémentaux permettent, grâce à la géométrie connue du bras robotisée et à la géométrie connue de l'outil, instrument et autre porté par le bras 3, de calculer la position cartésienne de l'extrémité distale du bras robotisé, la position cartésienne de l'extrémité de l'outil, instrument et autre et l'orientation de ce dernier dans l'espace.

Le bras tel que décrit reçoit un carénage approprié de manière à présenter un minimum de recoins afin d'éviter que des poussières ou des éléments pathogènes puissent s'y loger et prospérer.

Le caisson 7 comporte en partie inférieure des organes de roulement omnidirectionnels 8 tels que des roulettes portées chacune par une monture omnidirectionnelle possédant un axe vertical de rotation. Ces roulettes 8 et montures assurent un déplacement aisé sur le sol.

Le caisson 7 comporte des moyens d'immobilisation par rapport au sol pour interdire son déplacement en cours d'intervention chirurgicale.

Selon une première forme de réalisation, ces moyens d'immobilisation sont constitués par des organes de blocage, connus en soi, associés aux roulettes 8 et aux montures, interdisent lorsqu'ils sont actifs la rotation des roulettes autour de leur axe naturel de rotation et le pivotement de la monture autour de l'axe vertical. Le caisson se trouve ainsi immobilisé par rapport au sol.

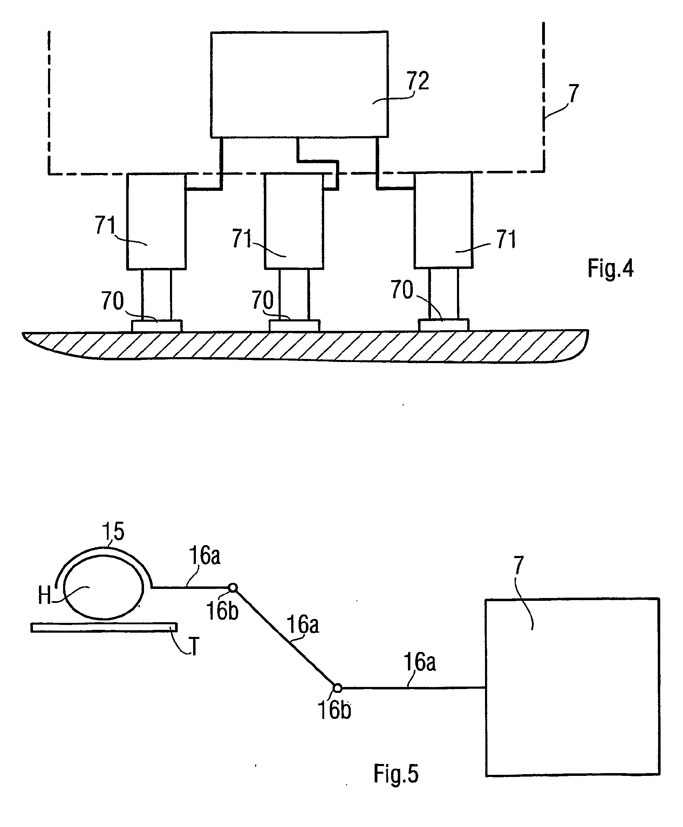

Selon une variante d'exécution, telle que représentée en figure 4, les moyens d'immobilisation du caisson 7 par rapport au sol, sont constitués par au moins trois pieds 70 pouvant occuper soit une position rétractée selon laquelle ils sont écartés du sol, soit une position déployée selon laquelle ils viennent en appui sur le sol pour immobiliser le caisson, chaque pied 70 étant mû de l'une à l'autre position par un organe moteur 71.

Selon une forme particulière de réalisation, chaque pied est formé par la partie terminale de la tige d'un vérin hydraulique du type simple effet associé à un circuit hydraulique approprié 72 commun aux différents vérins, comportant notamment comme connu, au moins un distributeur hydraulique et une pompe hydraulique entraînée par un moteur électrique piloté par une télécommande. Le ou chaque distributeur sera à commande électrique par exemple. Le moteur 71 pourra être aussi constitué par un vérin électrique.

Ainsi, l'utilisateur peut descendre les pieds 70 pour stabiliser le caisson 7, sans effectuer d'effort particulier.

Le caisson 7 peut être doté de moyens de fixation à une table chirurgicale sur laquelle a été préalablement installé le patient à opérer, la tête H de ce dernier étant maintenue fermement en position adéquate par une têtière fixe par rapport à la table.

Les moyens de fixation du caisson 7 à la table opératoire interdisent tout mouvement du caisson par rapport à la dite table et sont constitués par deux brides de fixation 9 aptes chacune à coopérer en fixation avec l'un des rails de la table, chaque bride étant portée de manière ajustable en position par une structure support 10 solidaire d'un rail support 11 porté par l'un des flancs du caisson 7. Chaque bride de fixation 9 présente des formes et dimensions compatibles avec celles des rails de la table. Chaque structure support 10 est constituée par un bras vertical 10a recevant la bride de fixation 9 correspondante. Cette bride 9 est montée avec une possibilité de déplacement en hauteur le long du bras vertical 10a et comporte un organe de serrage tel qu'une vis pression ou autre, d'immobilisation sur le bras 10a selon la position adéquate. Chaque structure support présente également un bras horizontal 10b fixé au bras vertical 10a. Ce bras horizontal 10b comporte un second bras vertical 10c engagé dans un coulisseau monté de manière ajustable en position dans le rail support 11 que présente le caisson 7. Ce second bras vertical sera immobilisé dans le coulisseau par une vis laquelle assure également l'immobilisation du coulisseau dans le rail support 11.

Selon une autre forme d'exécution, telle que représentée en figure 5, le caisson 7 n'est plus fixé à la table d'opération T mais directement à une têtière 15 prévue pour recevoir la tête H du patient à opérer, ce dernier étant installé sur table d'opération T. Ce caisson 7 est lié à la têtière 15 par l'entremise d'un moyen de fixation spécifique assurant, lorsqu'il est actif une liaison rigide entre le caisson 7 et la têtière 15. Cette disposition a l'avantage de s'affranchir de la flexibilité de la table opératoire T. Ces moyens de fixation peuvent être placés dans un état dit inactif autorisant le positionnement adéquat du caisson 7 par rapport à la têtière 15.

À titre d'exemple non limitatif, ce moyen peut être constitué par un bras mécanique 16 articulé, composé de plusieurs segments de bras 16a liés deux à deux par des articulations 16b associées à des brides d'immobilisation, non représentées, pouvant occuper chacune soit un état de verrouillage de l'articulation associée soit un état de déverrouillage de cette dernière.

Comme dit précédemment, au bras robot positionneur 3 sont associés une unité centrale 30 et une interface d'entrée informatique de données 32.

L'unité centrale des données 30 pourra être logée dans le caisson et former une partie de l'électronique que transporte ce dernier.

La plateforme robotisée peut comporter également un palpeur avec ou sans contact.

Le palpeur du type avec contact pourra être constitué par un pointeur mécanique porté par le segment distal terminal du bras de robot. Ce pointeur mécanique prévu pour être amené au contact de la cible à acquérir pourra comporter une bille de pointage ou une pointe sèche.

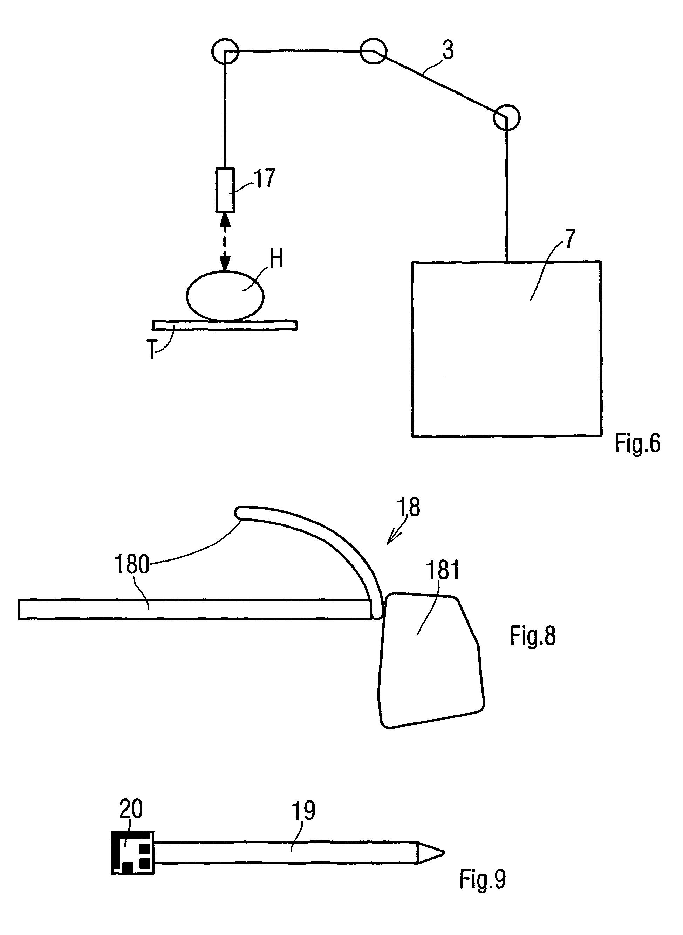

Un palpeur sans contact 17 constitué par un module optique de mesure de distance du genre télémètre laser selon l'invention, est représenté de manière schématique en figure 6 en association avec un bras robot 3. On observe sur cette figure que ce palpeur 15 est porté par le segment terminal distal de bras de robot et est orienté vers un point caractéristique de la tête H du patient.

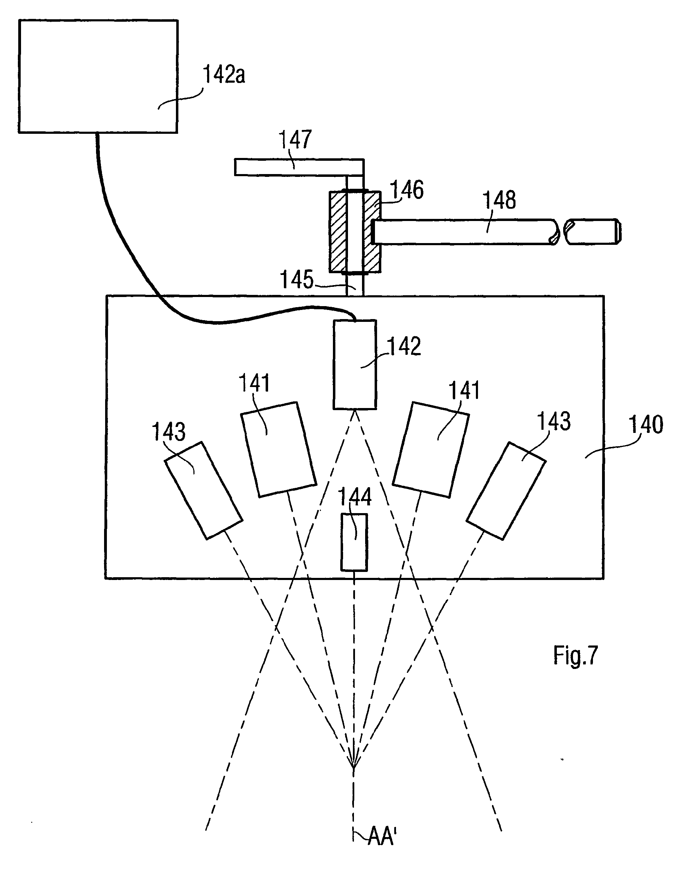

Le moyen de capture d'images 14 comporte au moins une caméra vidéo du type numérique par exemple. Dans une forme préférée de réalisation telle que représentée en figure 7, le moyen de capture d'images du champ opératoire comporte sur une platine support 140, deux caméras stéréoscopiques 141 par exemple du type numérique afin d'acquérir deux images vidéos stéréoscopiques de la zone anatomique à traiter et pouvoir restituer une vue 3D de ladite zone grâce à un système de visualisation d'images stéréoscopiques décrit ci-après. On remarque que ces deux caméras 141 sont disposées symétriquement par rapport un axe optique central AA'.

Ce moyen de capture d'images est doté de plus d'un module optique 142 adapté à être interfacé à un câble optique 143 lui-même connecté à une source de lumière froide 142a afin d'éclairer la zone anatomique à traiter.

Selon une disposition avantageuse de l'invention, le moyen de capture d'images 14 intègre deux modules lasers 143 latéraux aux caméras 141 et disposés de préférence symétriquement par rapport à l'axe optique AA'. Ces modules lasers 143 projettent des faisceaux lasers visibles et sont orientés en sorte que leur faisceau convergent en un point qui peut être réglé pour être le point d'intersection des deux axes optiques des caméras stéréoscopiques. Comme on peut le voir, le point de convergence appartient à l'axe optique AA'.

Ces modules lasers convergents 143 apportent un avantage en cours d'utilisation car ils indiquent la zone de travail optimale pour la perception du relief. Ils ont également un avantage en production pour faciliter le centrage des deux caméras stéréoscopiques sur le même point et déterminer la géométrie de l'outil moyen de capture d'images.

Le moyen de capture d'images 14 intègre de plus un module laser central 144 aligné sur l'axe optique central AA'. Ce faisceau laser visible matérialise l'axe dans lequel l'image vidéo de la zone anatomique est acquise.

Le module laser 144 est un télémètre laser apte à mesurer la distance entre sa face externe et l'objet le plus proche pointé par le faisceau laser.

Le moyen de capture d'images 14 pourra intégrer un système mécanique de rotation autour de son axe optique AA' permettant l'orientation de l'image vidéo formée sur les moyens de visualisation.

Un tel système de rotation pourra être réalisé par une liaison pivot ou pivot glissant avec l'ensemble des éléments décrits ci-dessus solidaires d'un arbre 145 engagé avec ajustement glissant dans l'alésage traversant d'un fourreau 147 doté d'un support 148 de fixation amovible à l'organe récepteur 5 que comporte le segment terminal distal de bras robotisé. Cet arbre 145 pourra être fixé rigidement à la platine 140.

Extérieurement au fourreau 146, l'arbre 145 recevra une poignée de manoeuvre en pivotement 147 par action sur laquelle, le moyen de capture d'images 14 pourra être orienté de manière adéquate. Pourra être adjoint au fourreau 146 un mécanisme de serrage à mâchoires par exemple, non représenté, prévu pour venir en serrage sur l'arbre 145 lorsqu'il est actif afin d'interdire tout mouvement de pivotement du moyen de capture 14 par rapport au fourreau 146. Ce mécanisme de mâchoires pourra être rendu inactif à partir de l'action sur une commande installée sur la poignée 147. À l'état inactif, ce mécanisme de serrage autorisera le pivotement du moyen de capture 14 par rapport au fourreau 146.

En figure 8 est représenté un exemple de réalisation d'un moyen 18 de visualisation du type trois dimensions. Ce moyen de visualisation stéréoscopique, prévu pour être porté par le chirurgien, comprend un casque 180 prévu pour être engagé sur la tête du chirurgien et de deux écrans de visualisation 181 à l'usage positionnables en regard des yeux du chirurgien. Sur les écrans de visualisation pourront s'afficher des images vidéo de la zone anatomique à traiter, éventuellement enrichies d'éléments virtuel de la planification afin d'assurer une fonction de réalité augmentée.

En figure 9 est représenté un pointeur mécanique 19 équipé d'au moins un marqueur visible 20, adapté à être repéré sur les images vidéo acquises par le moyen de capture d'images. On observe sur cette figure que le marqueur 20 comporte des zones fortement contrastée en l'espèce des zones blanches et des zones noires. Grâce au marqueur 20 la position spatiale de la pointe du pointeur 19 peut être calculée par triangulation par identification du marqueur sur les deux images stéréoscopiques fournies par les deux caméras vidéo du moyen de capture d'image 14.

La plate forme robotisée objet de la présente invention permet le positionnement d'un guide, d'un palpeur ou d'un moyen de capture d'images vidéos. Son utilisation repose sur quatre phases :
- un première phase d'acquisition des images numériques (scanner ou IRM) de la zone à traiter et de transfert de ces images numériques sur la console de planification 1 par le biais d'un réseau ou d'un support physique,
- une deuxième phase de traitement de ces images, d'identification de structures anatomiques et de planification de l'acte chirurgical, par exemple la définition d'un point d'entrée et d'un point cible pour établir une trajectoire d'une aiguille à biopsie,
- une troisième phase de mise en correspondance des images pré-opératoires avec la position de la tête du patient en per-opératoire selon l'un des modes décrits ci-après,
- et enfin une dernière étape de positionnement automatique d'un outil terminal 4, par exemple un guide, un pointeur laser, une caméra, un dispositif d'acquisition et autre.

La plateforme robotisée selon l'invention, reprend les mêmes hypothèses concernant l'affaissement du cerveau et la possible flexion des aiguilles, que les cadres stéréotaxiques, les systèmes de neuro-navigation et les systèmes robotiques.

La mise en correspondance entre les images pré-opératoires et la position du patient dans la salle opératoire peut être effectuée de plusieurs manières grâce aux différentes technologies intégrées dans la plateforme.

Selon une premier mode d'exécution, la méthode de recalage fait appel des marqueurs radio-opaques.

Lors de l'intervention, la mise en correspondance des images pré-opératoires (scanner ou IRM ou autre modalité) avec la position de la tête du patient peut être effectuée grâce à des marqueurs adaptés à être repérés sur l'imagerie, par exemple des marqueurs radio-opaques. Dans ce cas, lesdits marqueurs sont placés sur la tête du patient préalablement à l'acquisition des images pré-opératoires. Ils sont identifiés sur les images lors de la phase de planification afin de déterminer leur position dans le repère image (automatiquement avec une possibilité de retouche manuelle).

Lors de l'intervention, le chirurgien place le bras robotisé positionneur en mode coopératif et déplace manuellement ce bras robot positionneur équipé d'un palpeur afin de repérer les positions des différents marqueurs dans le repère robot. Une fois connues les positions des marqueurs dans le repère image et dans le repère robot, un algorithme de recalage point à point permet de mettre en correspondance les deux repères.

Cette méthode peut être effectuée avec les différents types de palpeur : l'instrument de pointage mécanique et le palpeur virtuel sans contact (télémètre laser).

Alternativement, le chirurgien place le bras robot équipé du moyen de capture d'images 14 au-dessus de la tête du patient. Le chirurgien peut alors procéder manuellement au repérage des marqueurs radio-opaques en utilisant le pointeur mécanique 19 équipé de marqueurs visibles 20 noir et blanc. Le système peut aussi procéder automatiquement au repérage des marqueurs radio-opaque en positionnant le moyen de capture d'images 14 dans différentes positions autour de la tête H du patient, acquérant des images stéréoscopiques contenant les marqueurs radio-opaques, segmentant les images pour repérer les marqueurs radio-opaques et calculant par triangulation leurs positions tridimensionnelles dans le système de coordonnées du bras robot. Afin de faciliter le repérage des marqueurs radio-opaques, des marqueurs spécifiques à fort contraste dans le visible peuvent être utilisés.

Une fois connues les positions des marqueurs dans le repère image et dans le repère robot, un algorithme de recalage point à point permet de mettre en correspondance les deux repères.

Selon une autre forme d'exécution, la méthode ne fait pas appel à des marqueurs radio-opaques.

Alternativement, la mise en correspondance des repères image et robot est effectuée à partir de repères anatomiques surfaciques en lieu et place des marqueurs. Lors de l'intervention, le chirurgien déplace manuellement le bras robot positionneur équipé d'un palpeur afin de repérer les positions de points ou surfaces anatomiques caractéristiques telles que nez, arcades, oreilles, dents ou autres. Un algorithme de recalage points-surface ou surface-surface permet de recaler les points ou surfaces ainsi acquises avec les examens pré-opératoires.

Cette méthode peut être effectuée avec les différents types de palpeur : l'instrument de pointage mécanique, le palpeur virtuel sans contact (télémètre laser), le pointeur mécanique équipé de marqueurs visibles noir et blanc.

Cette méthode de mise en correspondance présente l'avantage de ne pas nécessiter l'installation de marqueurs sur la tête du patient préalablement à l'imagerie.

Selon la méthode telle qu'exposée, le bras robot est déplacé manuellement par le chirurgien.

Alternativement, l'acquisition des surfaces anatomiques caractéristiques est effectuée sans contact et de façon automatique, en scannant tout ou partie de la tête H du patient. Une telle acquisition peut être obtenue par un capteur 4 de mesure sans contact par exemple un télémètre laser, fixé au bout du bras robot positionneur 3. Ledit bras robot scanne de façon automatique la zone d'intérêt en entraînant ledit capteur 4 selon un mouvement adapté en regard de ladite zone d'intérêt, par exemple, à vitesse constante selon un mouvement de translation rectiligne. La connaissance précise de la position du capteur 4 dans le repère robot permet la reconstruction des surfaces anatomiques.

Cette méthode peut être effectuée également avec le moyen de capture d'images lorsqu'il intègre un télémètre laser. Ceci est particulièrement avantageux lors d'une procédure de microscopie naviguée puisqu'il n'est plus nécessaire de changer l'outil en cours d'intervention.

Une sonde échographique peut également être utilisée en remplacement du capteur de mesure sans contact. Dans ce cas, la mise en correspondance des repères image et robot peut se faire grâce à un algorithme de traitement basé sur les propriétés de l'image telles qu'intensité, gradient, et autres propriétés.

Ces méthodes de mise en correspondance automatique ne nécessitent pas l'intervention manuelle du chirurgien.

Une fois la mise en correspondance effectuée, le robot positionne automatiquement l'outil fixé à l'organe récepteur sur la trajectoire planifiée.

Il va de soi que la présente invention peut recevoir toutes variantes du domaine des équivalents techniques sans pour autant sortir du cadre du présent brevet tel que défini par les revendications ci après.

## Revendications

1. Plateforme robotisée multi-applicative pour la neurochirurgie **caractérisée en ce qu'**elle comporte :
- une console de planification (1) intégrant des moyens de traitement (2) capables notamment de recevoir et traiter des images numériques,
- un bras robot positionneur (3) comportant plusieurs segments de bras dont un est terminal et proximal et l'autre terminal et distal, les dits segments étant liés les uns autres par des articulations et le segment distal terminal de bras comportant un organe de récepteur (5) agencé pour recevoir des outils (4), ledit bras robot (3) étant piloté par la console de planification (1),
- au moins un moyen (14) de capture d'images vidéo apte à capturer des images de la zone anatomique à traiter, ce dit moyen (14) pouvant être connecté électriquement aux moyens de traitement (2) que comporte la console de planification (1), et ce dit moyen de capture étant apte à être positionné et fixé de manière amovible à l'organe récepteur (5) du segment distal de bras, ce dit moyen de capture d'image comprenant un axe optique central (AA') et intégrant un module laser central (144) aligné sur l'axe optique central (AA'), ledit module laser central (144) étant un télémètre laser apte à mesurer la distance entre sa face externe et l'objet le plus proche pointé par le faisceau laser,
- des outils (4), instruments et autres, adaptés pour être positionnés et fixés de manière amovible à l'organe récepteur du segment distal terminal de bras,
des moyens de visualisation (6) des images pré-opératoires et per-opératoires, les dits moyens étant électriquement connectés à la console de planification (1) pour recevoir de cette dernière des signaux, vidéo propres aux images à visualiser, et/ou au moyen (14) de capture d'image.

2. Plateforme selon la revendication 1. **caractérisée en ce qu'**au robot positionneur (3) sont associés une unité centrale et une interface d'entrée informatique de données (32).

3. Plateforme selon l'une quelconque des revendications précédentes, **caractérisée en ce que** le bras robot positionneur (3) présente au moins six degrés de liberté à savoir trois translations et trois rotations.

4. Plateforme selon l'une quelconque des revendications précédentes, **caractérisée en ce que** le bras robot (3) comporte un capteur d'effort et est adapté à fonctionner selon un mode dans lequel un utilisateur a la capacité de déplacer le bras robot (3) manuellement en le saisissant par sa partie terminale.

5. Plateforme selon l'une quelconque des revendications précédentes, **caractérisée en ce qu'**elle est équipée d'un écran de contrôle (60) et d'une interface de communication (61) adaptée à recevoir d'un utilisateur des paramètres de planification opératoire.

6. Plateforme selon l'une quelconque des revendications précédentes, **caractérisée en ce que** les moyens de traitement (2) sont adaptés à définir chaque trajectoire grâce à des calculs tridimensionnels réalisés à partir des paramètres de planification opératoire et des coordonnées spatiales des repères anatomiques.

7. Plateforme selon l'une quelconque des revendications précédentes, **caractérisée en ce que** les outils (4) comprennent au moins un guide, et/ou au moins un pointeur mécanique, et/ou au moins un pointeur laser, et/ou au moins une sonde ultrasons, et/ou au moins un télémètre.

8. Plateforme Selon l'une que l'une quelconque des revendications 1 à 6 **caractérisée en ce que** les outils comprennent au moins un instrument chirurgical.

9. Plateforme selon l'une quelconque des revendications précédentes, **caractérisée en ce que** le bras robotisé positionneur (3) par son segment proximal terminal est fixé à une tourelle d'orientation installée fixement sur la partie supérieure d'un caisson parallélépipédique (7) comportant des organes de roulement au sol et des moyens d'immobilisation par rapport à ce dernier.

10. Plateforme selon la revendication précédente, **caractérisée en ce que** le caisson (7) est doté de moyens de fixation (9, 10, 11) à la table d'opération interdisant tout mouvement du caisson par rapport à ladite table.

11. Plateforme selon la revendication 9, **caractérisée en ce que** le caisson (7) comporte des moyens de fixation rigides à une têtière (15) installée sur la tête (H) d'un patient porté par la table d'opération (T).

12. Plateforme selon l'une quelconque des revendications précédentes, **caractérisée en ce que** le moyen (14) de capture d'images vidéo de la zone anatomique à traiter, comprend deux caméras stéréoscopiques (141) afin d'acquérir deux images vidéos stéréoscopiques de la zone anatomique à traiter et que les moyens de visualisation (6) comportent un système de visualisation d'images stéréoscopiques.

13. Plateforme selon la revendication 12, **caractérisée en ce que** le moyen de capture d'images (14) intègre un module optique (142) adapté à être interfacé à un câble optique lui-même connecté à une source de lumière froide (142a).

14. Plateforme selon la revendications 12 ou la revendication 13, **caractérisée en ce que** le moyen de capture d'images (14) intègre deux modules lasers (143) qui projettent des faisceaux lasers visibles convergent en un point apte à être réglé pour être le point d'intersection des deux axes optiques des caméras stéréoscopiques (141).

15. Plateforme selon l'une quelconque des revendications 12 à 14, **caractérisée en ce que** le moyen de capture d'images (14) intègre un système mécanique d'orientation (145, 146, 147, 148) par rotation selon son axe optique AA'.

16. Plateforme selon l'une quelconque des revendications précédentes, **caractérisée en ce** sur le ou l'un au moins des moyens de visualisation sont affichées des images vidéo de la zone anatomique à traiter augmentées d'éléments virtuels afin d'assurer une fonction de réalité augmentée.

17. Procédé de recalage de la zone anatomique à traiter par rapport à son modèle numérique et un bras robotisé (3) mettant en oeuvre une plateforme selon l'une quelconque des revendications précédentes, **caractérisé en ce qu'**il consiste :
- en l'acquisition antérieurement à l'intervention neurochirurgicale, de premières images numériques de la zone à traiter et au transfert de ces images numériques vers la console de planification par le biais d'un réseau ou d'un support physique en vue d'y être enregistrées et traitées,
- en l'acquisition automatique sans contact en per-opératoire, à l'aide d'un instrument de balayage porté par l'organe récepteur du segment distal terminal de bras robotisé, de secondes images numériques d'une partie pertinente d'une région corporelle du patient apparaissant déjà sur les premières images numériques, et au transfert de ces secondes images numériques vers la console de planification en vue d'y être enregistrées et traitées,
- en la constriction d'un premier modèle numérique en trois dimensions à partir des premières images numériques, ledit modèle faisant apparaître la région corporelle pertinente du patient,
- en la construction d'un second modèle numérique en trois dimensions à partir des secondes images numériques, faisant toujours apparaître la région corporelle pertinente du patient,
- et en la mise en correspondance des premier et second modèles par superposition des représentations de la région corporelle pertinente apparaissant sur l'un et l'autre modèle.

18. Procédé de recalage selon la revendication précédente, **caractérisé en ce que** pour la construction du second modèle numérique sont utilisées les données délivrées par le télémètre laser (144) et par les deux caméras 141 du moyen de captures d'images (14).

## Claims

1. Robotized multi-application platform for neurosurgery, wherein the platform includes:
- a planning console (1) integrating processing means (2) namely capable of receiving and processing digital images,
- a positioning robot arm (3) including several arm sections, one of which is terminal and proximal and the other one is terminal and distal, said sections being connected to each other by joints and the terminal distal arm section including a receiving organ (5) arranged for receiving tools (4), said robot arm (3) being driven by the planning console (1),
- at least one video image capturing means (14) capable of capturing images of the anatomic area to be processed, whereby said means (14) can be electrically connected to the processing means (2) the planning console (1) includes, and said capturing means is capable of being positioned and removably fixed to the receiving organ (5) of the distal arm section, this said image capturing means comprising a central optical axis (AA') and integrating a central laser module (144) aligned with the central optical axis (A A'), said central laser module (144) being a laser telemeter capable of measuring the distance between its external face and the closest object pointed to by the laser beam,
- tools (A), instruments and the like, adapted for being positioned and removably fixed to the receiving organ of the terminal distal arm section,
- means for displaying (6) the pre-operative and per-operative images, said means being electrically connected to the planning console (1) for receiving from the latter video signals specific to the images to be displayed, and/or to the image capturing means (14).

2. Platform according to claim 1, wherein with the positioning robot (3) are associated a central unit and a data-processing input interface (32).

3. Platform according to any of the preceding claims, wherein the positioning robot arm (3) has at least six degrees of freedom, namely three translations and three rotations.

4. Platform according to any of the preceding claims, wherein the robot arm (3) includes an effort sensor and is adapted for operating according to a mode in which the user has the capability of displacing the robot arm (3) manually by seizing it by its terminal portion.

5. Platform according to any of the preceding claims, wherein the platform is provided with a control screen (60) and a communication interface (61) adapted for receiving operative planning parameters from a user.

6. Platform according to any of the preceding claims, wherein the processing means (2) are adapted for defining each path thanks to three-dimensional calculations performed based on the operative planning parameters and the spatial coordinates of the anatomic marks.

7. Platform according to any of the preceding claims, wherein the tools (4) comprise at least one guide, and/or at least one mechanical pointer, and/or at least one laser pointer, and/or at least one ultrasound probe, and/or at least one telemeter.

8. Platform according to any of claims 1 to 6, wherein the tools comprise at least one surgical instrument.

9. Platform according to any of the preceding claims, wherein the robotized positioning arm (3) is fixed through its terminal proximal section to an orientation turret firmly installed on the upper portion of a parallelepipedal case (7) including organs for rolling on the ground and means for immobilizing with respect to the latter.

10. Platform according to any of the preceding claim, wherein the case (7) is provided with means for fastening (9, 10, 11) to the operation table impeding any movement of the case with respect to said table.

11. Platform according to claim 9, wherein the case (7) includes means for firmly fastening to a face-plate (15) installed on the head (H) of a patient carried by the operation table (T).

12. Platform according to any of the preceding claims, wherein the means (14) for capturing the video images of the anatomic area to be treated comprises two stereoscopic cameras (141) in order to acquire two stereoscopic video images of the anatomic area to be treated and the display means (6) include a stereoscopic image display system.

13. Platform according to claim 12, wherein the image capturing means (14) integrates an optical module (142) adapted for being interfaced with an optical cable, which is in turn connected to a cold light source (142a).

14. Platform according to claim 12 or claim 13, wherein the image capturing means (14) integrates two laser modules (143), which project visible laser beams converging at a point capable of being adjusted so as to be the point of intersection of two optical axes of the stereoscopic cameras (141).

15. Platform according to any of claims 12 to 14, wherein the image capturing means (14) integrates a system for mechanical orientation (145, 146, 147, 148) by rotation according to its optical axis (AA').

16. Platform according to any of the preceding claims, wherein on the or on at least one of the display means are displayed video images of the anatomic area to be treated increased with virtual elements in order to ensure an increased reality function.

17. Method for setting the anatomic area to be treated with respect to its digital model and a robotized arm (3) implementing a platform according to any of the preceding claims, wherein the method consists:
- in acquiring, prior to the neurosurgical intervention, first digital images of the area to be treated and in transferring these digital images to the planning console through a network or a physical carrier in order to be stored and processed on same,
- in automatically acquiring contactless in per-operative phase, by means of a scanning instrument carried by the receiving organ of the terminal distal section of the robotized arm, second digital images of a relevant portion of a body area of the patient already appearing in the first digital images, and in transferring these second digital images to the planning console in order to be stored and processed on same,
- in construing a first three-dimensional digital model based on the first digital images, said model showing the relevant body region of the patient,
- in construing a second three-dimensional digital model based on the second digital images, also showing the relevant body region of the patient,
- in putting in correspondence the first and second models by superposing the representations of the relevant body region appearing in either model.

18. Method for resetting according to the preceding claim, wherein the data provided by the laser telemeter (144) and by the two cameras 141 of the image capturing means (14) are used for construing the second digital model.

## Patentansprüche

1. Roboterisierte Mehranwendungs-Plattform für Neurochirurgie, **dadurch gekennzeichnet, dass** die Plattform Folgendes umfasst:
- eine Planungskonsole (1), die Verarbeitungsmittel (2) integriert, die nämlich geeignet sind, um digitale Bilder zu empfangen und zu verarbeiten,
- einen Positionierungs-Roboterarm (3), der mehrere Armabschnitte umfasst, von denen einer terminal und proximal und der andere terminal und distal ist, wobei die besagten Abschnitte über Gelenke mit einander verbunden sind und der terminale distale Armabschnitt ein Aufnahmeorgan (5) umfasst, das zum Aufnehmen von Werkzeugen (4) eingerichtet ist, wobei der besagte Roboterarm (3) von der Planungskonsole (1) getrieben wird,
- wenigstens ein Videobilderfassungsmittel (14), das geeignet ist, um Bilder des zu verarbeitenden anatomischen Bereichs zu erfassen, wobei dieses besagte Mittel (14) elektrisch mit den Verarbeitungsmitteln (2), welche die Planungskonsole (1) umfasst, verbunden sein können, und das besagte Bilderfassungsmittel geeignet ist, um an das Aufnahmeorgan (5) des distalen Armabschnitts positioniert und entfernbar befestigt zu werden, wobei das besagte Bilderfassungsmittel eine zentrale optische Achse umfasst (AA') und ein zentrales Lasermodul (144) integriert, das auf der zentralen optischen Achse (AA') ausgerichtet ist, wobei das zentrale Lasermodul (144) ein Lasertelemeter ist, das geeignet ist, um den Abstand zwischen seiner Außenfläche und dem am nächsten liegenden, vom Laserstrahl gezeigten Gegenstand zu messen,
- Werkzeuge (A), Instrumente und dergleichen, die angepasst sind, um an das Aufnahmeorgan des terminalen distalen Armabschnittes positioniert und entfernbar befestigt zu werden,
- Mittel zum Anzeigen (6) der prä-operative und per-operative Bilder, wobei die besagten Mittel elektrisch mit der Planungskonsole (1), um von der letzteren anzuzeigenden, den Bildern eignen Videosignale zu erhalten, und/oder mit dem Bilderfassungsmittel (14) verbunden sind.

2. Plattform nach Anspruch 1, **dadurch gekennzeichnet, dass** dem Positionierungsroboter (3) eine Zentraleinheit und eine Datenverarbeitungs-Eingabeschnittstelle zugeordnet sind (32).

3. Plattform nach irgendeinem der vorgehenden Ansprüche, **dadurch gekennzeichnet, dass** der Positionierungsroboterarm (3) wenigstens sechs Freiheitsgrade, nämlich drei Translationen und drei Drehungen, aufweist.

4. Plattform nach irgendeinem der vorgehenden Ansprüche, **dadurch gekennzeichnet, dass** der Roboterarm (3) einen Kraftgeber umfasst und angepasst ist, um nach einer Betriebsart zu arbeiten, in dem der Gebraucher die Möglichkeit hat, den Roboterarm (3) manuell zu verschieben, indem er diesen an seinem Terminalteil ergreift.

5. Plattform nach irgendeinem der vorgehenden Ansprüche, **dadurch gekennzeichnet, dass** sie mit einem Kontrollschirm (60) und einer Kommunikationsschnittstelle (61) versehen ist, die angepasst ist, um von dem Gebraucher operative Planungsparameter zu empfangen.

6. Plattform nach irgendeinem der vorgehenden Ansprüche, **dadurch gekennzeichnet, dass** die Verarbeitungsmittel (2) angepasst sind, um jeden Weg dank dreidimensionaler Berechnungen zu definieren, die auf Basis der operativen Planungsparameter und der räumlichen Koordinaten der anatomischen Markierungen durchgeführt werden.

7. Plattform nach irgendeinem der vorgehenden Ansprüche, **dadurch gekennzeichnet, dass** die Werkzeuge (4) wenigstens eine Führung, und/oder wenigstens einen mechanischen Zeiger, und/oder wenigstens einen Laserzeiger, und/oder wenigstens einen Ultraschallfühler, und/oder wenigstens ein Telemeter umfassen.

8. Plattform nach irgendeinem der Ansprüche 1 bis 6, **dadurch gekennzeichnet, dass** die Werkzeuge wenigstens ein chirurgisches Instrument umfassen.

9. Plattform nach irgendeinem der vorgehenden Ansprüche, **dadurch gekennzeichnet, dass** der roboterisierte Positionierungsarm (3) mit seinem terminalen proximalen Abschnitt an einem Orientierungsturm befestigt ist, der fest auf dem oberen Teil eines quaderförmigen Kastens (7) aufgestellt ist, der Organe zum Rollen auf dem Boden und Mittel zur Immobilisierung bezüglich dieses letzteren umfasst.

10. Plattform nach irgendeinem der vorgehenden Ansprüche, **dadurch gekennzeichnet, dass** der Kasten (7) mit Mitteln zur Befestigung (9, 10, 11) an den Operationstisch versehen ist, die jegliche Bewegung des Kastens bezüglich des besagten Tisches verhindert.

11. Plattform nach Anspruch 9, **dadurch gekennzeichnet, dass** der Kasten (7) Mittel zur festen Befestigung an eine Stulpe (15), die auf dem Kopf (H) eines von dem Operationstisch (T) getragenen Patienten installiert ist.

12. Plattform nach irgendeinem der vorgehenden Ansprüche, **dadurch gekennzeichnet, dass** das Mittel (14) zum Erfassen der Videobilder des zu behandelnden anatomischen Bereichs zwei stereoskopische Kameras (141) umfasst, um zwei stereoskopische Videobilder des zu behandelnden anatomischen Bereichs zu erfassen, und die Anzeigemittel (6) ein stereoskopisches Bildanzeigesystem umfassen.

13. Plattform nach Anspruch 12, **dadurch gekennzeichnet, dass** das Bilderfassungsmittel (14) ein optisches Modul (142) integriert, das angepasst ist, um als Schnittstelle mit einem optischen Kabel verbunden zu werden, das seinerseits mit einer Kaltlichtquelle (142a) verbunden ist.

14. Plattform nach Anspruch 12 oder Anspruch 13, **dadurch gekennzeichnet, dass** das Bilderfassungsmittel (14) zwei Lasermodule (143) integriert, die sichtbare Laserstrahle ausstrahlen, die an einem Punkt konvergieren, der geeignet ist, um so eingestellt zu werden, dass er der Schnittpunkt zweier optischen Achsen der stereoskopischen Kameras (141) ist.

15. Plattform nach irgendeinem der Ansprüche 12 bis 14, **dadurch gekennzeichnet, dass** das Bilderfassungsmittel (14) ein System zur mechanischen Orientierung (145, 146, 147, 148) durch Drehen gemäß seiner optischen Achse (AA') integriert.

16. Plattform nach irgendeinem der vorgehenden Ansprüche, **dadurch gekennzeichnet, dass** auf dem oder auf wenigstens einem der Anzeigemittel Videobilder des zu behandelnden anatomischen Bereichs angezeigt werden, erhöht um virtuelle Elemente, um eine erhöhte Echtheitsfunktion zu sichern.

17. Verfahren zum Einstellen des zu behandelnden anatomischen Bereichs bezüglich des digitalen Modells desselben und eines roboterisierten Armes (3), das eine Plattform nach irgendeinem der vorgehenden Ansprüche anwendet, **dadurch gekennzeichnet, dass** es darin besteht:
- vor dem neurochirurgischen Eingriff erste digitale Bilder des zu behandelnden Bereichs zu erfassen und diese digitalen Bilder über ein Netz oder einen physischen Träger an die Planungskonsole zu senden, um darauf gespeichert und verarbeitet zu werden,
- in der per-operative Phase automatisch und berührungslos zweite digitale Bilder eines relevanten Teils eines Körperbereichs des Patienten, der schon in den ersten digitalen Bilder erscheint, mittels eines Abtastinstruments, das von dem Aufnahmeorgan des terminalen distalen Abschnitts des roboterisierten Armes getragen wird, zu erfassen und diese zweiten digitalen Bilder an die Planungskonsole zu senden, um darauf gespeichert und verarbeitet zu werden,
- ein erstes dreidimensionales Modell auf Basis der ersten digitalen Bilder aufzubauen, wobei das besagte Modell den relevanten Körperbereich des Patienten zeigt,
- ein zweites dreidimensionales Modell auf Basis der zweiten digitalen Bilder aufzubauen, das ebenfalls den relevanten Körperbereich des Patienten zeigt,
- das erste und das zweite Modell in Übereinstimmung zu bringen, indem die Darstellungen des relevanten Körperbereiches, der in dem einen und dem anderen Modell erscheint, über einander gesetzt werden.

18. Verfahren zum Einstellen nach dem vorgehenden Anspruch, **dadurch gekennzeichnet, dass** zum Aufbauen des zweiten digitalen Modells von dem Lasertelemeter (144) und von den zwei Kameras 141 des Bilderfassungsmittel (14) gelieferte Daten verwendet werden.
